# EUROPÄISCHE PATENTANMELDUNG

(11) **EP 4 711 135 A1**
(43) Veröffentlichungstag der Anmeldung: **18.03.2026**
(21) Anmeldenummer: 25200866.9
(22) Anmeldetag: 08.09.2025
(51) Int. Cl.: B33Y 80/00, A61F 2/38, A61F 2/30

(54) **TIBIA-IMPLANTAT**

(30) Priorität: 11.09.2024 DE 102024126035
(71) Anmelder: Aesculap AG, 78532 Tuttlingen (DE)
(72) Erfinder: Richter, Berna, 78570 Mühlheim (DE); Dohm, Julius, 61476 Kronberg (DE); Grupp, Thomas, 78588 Denkingen (DE)
(74) Vertreter: DREISS Patentanwälte PartG mbB

(57) **Zusammenfassung**

Die Erfindung betrifft ein Tibia-Implantat (2) für Gelenkersatz, welches aus Metalllegierung in einem additiven Fertigungsverfahren, insbesondere durch metallischen 3D-Druck, hergestellt ist, mit einem Plateauabschnitt (4) und mit einem auf der tibiazugewandten Seite (8) des Plateauabschnitts (4) vorspringenden und sich davon in einer axialen Richtung (10) wegerstreckenden zapfen- oder kielförmigen Ankerabschnitt (6), welcher in einen hierfür vorbereiteten Kanal in einem Tibiaknochen in der axialen Richtung (10) einsetzbar ist; erfindungsgemäß wird vorgeschlagen, dass der Plateauabschnitt (4) auf seiner tibiazugewandten und mit Tibiaknochengewebe in Kontakt gelangenden Seite (8) eine dreidimensional poröse offenporige Oberflächenstruktur (14) mit in der axialen Richtung (10) hintergreifbaren Brücken, Stegen oder Wandbereichen aufweist, wobei die dreidimensional poröse offenporige Oberflächenstruktur (14) eine erste Rauigkeit aufweist, und dass der zapfen- oder kielförmige Ankerabschnitt (6) anschließend an den Plateauabschnitt (4) in einem ersten axialen Ankerbereich (20) umfangsseitig eine in einer radialen Richtung (12) hinterschnittfreie, das heißt von hintergreifbaren Brücken und brückenbildenden Stegen freie Oberflächenstruktur (28) mit einer zweiten Rauigkeit aufweist, die geringer ist als die erste Rauigkeit, und
dass der zapfen- oder kielförmige Ankerabschnitt (6) in der axialen Richtung (10) anschließend an den ersten axialen Ankerbereich (20) einen zweiten axial frei endenden axialen Ankerbereich (22) mit einer glatten Oberfläche aufweist.

## Beschreibung

Die Erfindung betrifft ein Tibia-Implantat für Gelenkersatz, welches aus Metalllegierung in einem additiven Fertigungsverfahren, insbesondere nach Norm ASTM 52900:2022-03, insbesondere durch metallischen 3D-Druck, hergestellt ist, mit einem Plateauabschnitt, an welchem tibiaabgewandt ein Lagerbereich für artikulierende kondyläre Gelenkoberflächen vorgesehen und insbesondere ein Meniskusersatzteil anordenbar ist, und mit einem auf der tibiazugewandten Seite des Plateauabschnitts vorspringenden und sich davon in einer axialen Richtung wegerstreckenden zapfen- oder kielförmigen Ankerabschnitt, welcher in einen hierfür vorbereiteten Kanal in einem Tibiaknochen in der axialen Richtung einsetzbar ist.

Auf dem hier in Rede stehenden Gebiet unterscheidet man zwischen solchen Implantaten, welche typischerweise in einen hierfür vorbereiteten Kanal in den Tibiaknochen einzementiert werden und solchen, welche zementfrei eingesetzt und schließlich durch Anwachsen von Tibiaknochengewebe im Tibiaknochen verankert werden. Naturgemäß soll nach beiden Methoden ein dauerhafter Festsitz des Tibia-Implantats im Tibiaknochen erreicht werden. Es kann aber auch in Einzelfällen erforderlich werden, das Implantat wieder aus dem Tibiaknochen herauszulösen und durch ein neues zu ersetzen.

Zur Erzielung eines Festsitzes sowohl bei einzuzementierenden Implantaten als auch bei zementfrei einzubringenden Implantaten wurden zahlreiche Vorschläge zur Ausgestaltung der verschiedenen Oberflächenbereiche eines Implantats unterbreitet. Beispielsweise wird mit US 2024/0041605 A1 vorgeschlagen, beim Schaft eines Gelenkimplantats verschieden poröse und verschieden raue Umfangsbereiche mit hintergreifbaren Gitterstrukturen vorzusehen.

Der vorliegenden Erfindung liegt die Aufgabe zugrunde, ein Tibia-Implantat der eingangs genannten Art dahingehend weiterzuentwickeln, dass sich auch im Wege einer zementfreien Implantation ein dauerhafter Festsitz im Tibiaknochen erreichen lässt, wobei aber im erwünschten Fall auch eine Implantatentfernung möglich sein soll, ohne den Tibiaknochen dabei zu schädigen.

Zur Lösung dieser Aufgabe wird ausgehend von einem Tibia-Implantat der genannten Art erfindungsgemäß vorgeschlagen, dass der Plateauabschnitt auf seiner tibiazugewandten und mit Tibiaknochengewebe in Kontakt gelangenden Seite eine dreidimensional poröse offenporige Oberflächenstruktur mit in der axialen Richtung hintergreifbaren Brücken, Stegen oder Wandbereichen aufweist, wobei die dreidimensional poröse offenporige Oberflächenstruktur eine erste Rauigkeit aufweist, dass der zapfen- oder kielförmige Ankerabschnitt anschließend an den Plateauabschnitt in einem ersten axialen Ankerbereich umfangsseitig eine in einer radialen Richtung hinterschnittfreie, das heißt von hintergreifbaren Brücken und brückenbildenden Stegen freie Oberflächenstruktur mit einer zweiten Rauigkeit aufweist, die geringer ist als die erste Rauigkeit, und
dass der zapfen- oder kielförmige Ankerabschnitt in der axialen Richtung anschließend an den ersten axialen Ankerbereich einen zweiten axial frei endenden axialen Ankerbereich mit einer glatten Oberfläche aufweist.

Durch die Ausgestaltung der tibiazugewandten Seite des Plateauabschnitts mit einer Oberflächenstruktur mit in der axialen Richtung hintergreifbaren Brücken, Stegen oder Wandbereichen wird die Möglichkeit geschaffen, dass dort Tibiaknochengewebe nicht nur an die metallische Oberfläche anwächst, sondern in die dreidimensional poröse Oberflächenstruktur einwächst und somit den Implantatkörper in der Implantationsrichtung, also in der axialen Richtung, fest am Tibiaknochen hält. Im Extraktionsfall kann dort im wesentlichen in radialer Richtung, also orthogonal zur axialen oder Transplantationsrichtung, ein Resektionsschnitt mittels eines Sägeblatts ausgeführt werden, um diese stabile Verbindung zum Extrahieren des Implantats zu durchtrennen, was in einer OP-Situation ohne weiteres möglich ist.

Dadurch, dass axial anschließend an den Plateauabschnitt in dem ersten axialen Ankerbereich umfangsseitig eine in der radialen Richtung hinterschnittfreie Oberflächenstruktur, also eine von hintergreifbaren Brücken oder brückenbildenden Stegen freie Oberflächenstruktur, vorgesehen wird, deren zweite Rauigkeit geringer ist als die erste Rauigkeit der vorerwähnten dreidimensional porösen Oberflächenstruktur, vermag das sich dann bildende Tibiaknochengewebe auch nur an diese umfangsseitige Oberflächenstruktur anzuwachsen; in Ermangelung einer dreidimensionalen Porosität und von hintergreifbaren Brücken und Stegen ist ein Einwachsen in hintergreifbare Bereiche, Tunnel oder Pfade hingegen nicht möglich. Es wurde festgestellt, dass sich anwachsendes Knochengewebe von in poröse Strukturen einwachsendem Knochengewebe von seiner biologischen Struktur her unterscheidet und dass sich ersteres bei Scherbeanspruchung leichter lösen lässt als letzteres. Dennoch vermag auch an hinterschnittfreie Oberflächenstrukturen lediglich anwachsendes Knochengewebe eine beträchtliche Haftkraft und dauerhafte Verbindung mit der Struktur auszubilden, so dass auch hierdurch ein dauerhafter Festsitz erreicht werden kann, welcher den im Betrieb des Implantats auftretenden Belastungen standzuhalten vermag, ohne dass sich das Implantat des Gelenkersatzes löst.

Es wurde weiter festgestellt, dass es vorteilhaft ist, wenn ein zweiter distaler Ankerbereich, der sich in der axialen Richtung an den ersten axialen Ankerbereich anschließt, glatt ausgebildet ist, weil hierdurch das Einführen des Ankerabschnitts in den hierfür vorbereiteten Kanal in dem Tibiaknochen erleichtert ist; außerdem wird durch diese oberflächenglatte Ausbildung beim Einführen des Ankerabschnitts in den vorbereiteten Kanal kein Tibiaknochengewebe abgeraspelt und somit die Maßhaltigkeit des Kanals nicht in unvorhersehbarer Weise verändert. Des Weiteren zeigte sich, dass sich das Implantat bei dieser glatten Ausbildung in dem zweiten axialen Ankerbereich erwünschtenfalls auch atraumatischer wieder entfernen lässt.

Wenn in der vorliegenden Anmeldung von einer glatten Ausbildung einer Oberfläche bei dem Implantat oder einem Implantatbereich die Rede ist, so bedeutet dies, dass dort keine dreidimensional variierende Oberflächenstruktur mit Erhebungen, Vertiefungen oder Stegen durch das additive Fertigungsverfahren, insbesondere durch 3D-Druck, aufgebaut ist, sondern dass das additive Fertigungsverfahren volumenfüllend ausgeführt wurde, so dass eine geschlossene Oberfläche entsteht, welche lediglich eine der additiven Fertigung inhärente geringe Oberflächenrauigkeit aufweist, die demgemäß auch deutlich geringer ist als die zweite Rauigkeit des ersten axialen Ankerbereichs. Im Anschluss an die additive Fertigung kann die glatte gleichförmige Oberfläche zudem poliert werden, sofern dies gewünscht sein sollte.

Des Weiteren sei erläutert, dass eine Oberfläche weniger rau im Sinne der vorliegenden Anmeldung ist als eine andere Oberfläche, wenn sie in Bezug auf den Rauigkeitsparameter Ra bestimmt nach DIN/ISO 21920-3 als weniger rau zu bezeichnen ist als eine andere Oberfläche.

Es erweist sich weiter als vorteilhaft, wenn die dreidimensional poröse Oberflächenstruktur eine Tiefenerstreckung ausgehend von einer die Oberflächenstruktur einhüllenden Oberfläche oder Hüllfläche von wenigstens 1,5 mm, insbesondere von wenigstens 1,8 **mm,** insbesondere von wenigstens 2,0 **mm,** insbesondere von wenigstens 2,5 mm**,** insbesondere von höchstens 4,0 **mm,** insbesondere von höchstens 3,8 **mm,** insbesondere von höchstens 3,5 **mm,** insbesondere von höchstens 3,0 mm aufweist. Bevorzugt wird eine Tiefenerstreckung von 2,0 bis 3,5 mm**.** Innerhalb der genannten Bereiche kann eine zufriedenstellende zementfreie Verankerung des Plateauabschnitts des Tibia-Implantats durch Einwachsen von Knochengewebe in die dreidimensional poröse offenporige Oberflächenstruktur erreicht werden.

Es erweist sich weiter als vorteilhaft und durch additive Fertigungstechnik ohne weiteres realisierbar, dass die dreidimensional poröse offenporige Oberflächenstruktur von einer zusammenhängenden ein dreidimensionales Gitter bildenden Stegstruktur gebildet ist und dass ein Stegdurchmesser wenigstens 0,5 mm, insbesondere wenigstens 0,6 mm insbesondere wenigstens 0,7 mm, insbesondere höchstens 1,1 mm, insbesondere höchstens 1,0 mm, insbesondere höchstens 0,9 mm beträgt.

Weiter erweist es sich als vorteilhaft, wenn die dreidimensional poröse offenporige Oberflächenstruktur eine Porengröße von wenigstens 0,8 mm, insbesondere von wenigstens 0,9 mm, insbesondere von wenigstens 1,0 mm, insbesondere von höchstens 1,4 mm, insbesondere von höchstens 1,3 mm, insbesondere von höchstens 1,2 mm aufweist, wobei es sich hierbei um den Durchmesser einer Kugel handelt, die in den Poren aufnehmbar ist.

In weiterer Ausbildung der Erfindung erweist es sich als vorteilhaft, wenn die in radialer Richtung hinterschnittfreie umfangsseitige Oberflächenstruktur des ersten axialen Ankerbereichs eine deutlich geringere Tiefenerstreckung ausgehend von einer die Oberflächenstruktur einhüllenden Oberfläche oder Hüllfläche aufweist als die dreidimensional poröse offenporige Oberflächenstruktur bei dem Plateauabschnitt. Sie beträgt ausgehend von einer die Oberflächenstruktur einhüllenden Oberfläche wenigstens 0,2 mm, insbesondere wenigstens 0,3 mm und höchstens 0,9 mm, insbesondere höchstens 0,8 mm, insbesondere höchstens 0,7 mm aufweist.

Es erweist sich weiter als vorteilhaft, wenn die in radialer Richtung hinterschnittfreie umfangsseitige Oberflächenstruktur des ersten axialen Ankerbereichs sich von einer Grundfläche des Ankerbereichs erhebende Strukturen aufweist. Hierbei kann die Grundfläche eine gegenüber den sich hiervon erhebenden Strukturen glatte Umfangsfläche des ersten axialen Ankerbereichs bilden, von der sich dann diese Strukturen wie Bergstrukturen erheben.

Weiter kann es vorteilhaft sein, wenn die in radialer Richtung hinterschnittfreie Oberflächenstruktur des ersten axialen Ankerbereichs bei Betrachtung in radialer Richtung hin- und her mäandrierende, sich erhebende Strukturen aufweist.

Es kann sich auch als vorteilhaft erweisen, wenn die sich erhebenden Strukturen eine Vielzahl von ebenen Facettenflächen umfassen, die über Kanten aneinander angrenzen. Insbesondere können die sich erhebenden Strukturen vollständig von solchen ebenen Facettenflächen gebildet oder begrenzt sein. Das Anwachsen von Knochengewebe an ebenen Facettenflächen bewährt sich nämlich bei der hier in Rede stehenden Anwendung.

Weiter bewährt es sich, wenn die mäandrierenden sich erhebenden Strukturen im Wesentlichen ebene oder glatte Bereiche einer Grundfläche zwischen sich begrenzen.

Weiter erweist es sich als vorteilhaft, wenn benachbarte mäandrierende sich erhebende Strukturen einander berühren und zwischen sich erhebungsfreie oder weniger erhabene Gebiete begrenzen. Die sich erhebenden Strukturen bilden dann bei Ansicht in radialer Richtung und regelmäßiger periodischer Anordnung und Ausbildung ein ebenes netzartiges Gebilde, dessen Öffnungen durch die erhebungsfreien oder weniger erhabenen Gebiete gebildet ist.

Es kann sich aber auch als vorteilhaft erweisen, wenn benachbarte mäandrierende sich erhebende Strukturen voneinander beabstandet sind, so dass zwischen ihnen streifenförmig zusammenhängende erhebungsfreie oder weniger erhabene Gebiete begrenzt werden.

Weiter kann sich aber auch als vorteilhaft erweisen, wenn die in radialer Richtung hinterschnittfreie Oberflächenstruktur des ersten axialen Ankerbereichs eine Vielzahl von einzelnen inselartig voneinander beabstandeten sich erhebenden Strukturen aufweist. Solchenfalls werden zwischen diesen Strukturen zusammenhängende erhebungsfreie oder weniger erhabene Gebiete gebildet oder begrenzt.

Weiter wird vorgeschlagen, dass erhebungsfreie oder weniger erhabene Gebiete zwischen sich erhebenden Strukturen glatt ausgebildet sind. Solchenfalls sind also die sich erhebenden Strukturen wie Bergstrukturen ausgebildet, die sich von ebenen Tälern zwischen ihnen erheben.

Als Material für zementfreie Implantate wurde seither typischerweise Kobalt-Chrom-Legierung verwendet, die sich aber im Hinblick auf die Verträglichkeit als problematisch erweist. Insoweit ist es vorteilhaft, dass festgestellt wurde, dass sich ein Implantat der vorliegend beanspruchten Art durch Titan- oder Titanlegierung fertigen lässt und dass dennoch sowohl das Einwachsen von Knochengewebe in hintergreifbare Strukturen, Poren, Tunnel, Pfade einerseits als auch das Anwachsen von Knochengewebe an weniger komplexe Oberflächenstrukturen ohne Brücken und Tunnel zu einem hinreichenden Festsitz führt.

In Weiterbildung der Erfindung ist es ferner möglich, dass nicht mit Tibiaknochengewebe in Kontakt stehende Oberflächenbereiche des Plateauabschnitts, also in Weichteilkontakt stehende Umfangsbereiche oder mit einem Meniskusersatzteil in Kontakt stehende Oberflächenbereiche, glatt sind. Es wurde nämlich festgestellt, dass in Weichteilkontakt stehende Implantatoberflächen zu problematischen Gewebereizungen führen und sich somit sehr negativ auswirken können. Insofern wird außerdem vorgeschlagen, dass nicht mit Tibiaknochengewebe in Kontakt stehende glatte Oberflächenbereiche des Plateauabschnitts zumindest bereichsweise eine Beschichtung aufweisen, mittels derer eine Oberflächenrauheit dieser glatten Oberflächenbereiche weiter herabgesetzt wird. Eine solche Beschichtung insbesondere von in Weichteilkontakt stehenden Oberflächenbereichen von Kniegelenkimplantaten wurde in der Patentanmeldung DE 2023 114 759 der Anmelderin beschrieben. Die Ausbildung, insbesondere die mehrlagige Ausbildung, dieser Beschichtung ist in dieser nicht vorveröffentlichten Patentanmeldung DE 2023 114 759 erläutert, so dass für Offenbarungszwecke der diesbezügliche Inhalt durch Verweis in die vorliegende Anmeldung einbezogen wird:
Danach erweist es sich insbesondere als vorteilhaft, wenn die Beschichtung eine keramische Oberfläche, insbesondere aus oder auf Basis von Zirkonnitrid, umfasst.

Weiter erweist es sich als vorteilhaft, wenn die Beschichtung mehrlagig ausgebildet ist und über eine Haftvermittlungschicht, insbesondere auf Kobalt-Chrom-Basis oder auf Titan-Basis, an das Implantatteil angebunden ist.

Weiter erweist es sich als vorteilhaft, wenn die Beschichtung Schichten auf Basis von Kobalt-Chrom und/oder auf Basis von Chromnitrid (CrN) und/oder auf Basis von Chromcarbonitrid (CrCN) und/oder auf Basis von Zirkoniumnitrid (ZrN) umfasst.

Weiter erweist es sich als vorteilhaft, wenn die Beschichtung eine Deckschicht auf Basis von Zirkonnitrid (ZrN) und innere Schichten auf Basis von Chromnitrid (CrN) oder Chromcarbonitrid (CrCN)aufweist, wobei sich innere Schichten auf Basis von Chromnitrid (CrN) und Chromcarbonitrid (CrCN) abwechseln können.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen Tibia-Implantats.

In der Zeichnung zeigt:
Figur 1 eine perspektiviche Ansicht eines erfindungsgemäßen Tibia-Implantats;
Figur 2 verdeutlicht eine dreidimensional porse Oberflächenstruktur auf der tibiazugewandten Seite eines Plateauabschnitts des Tibia-Implantats nach Figur 1;
Figur 3 verdeutlicht schematisch eine in radialer Richtung hinterschnittfreie Oberflächenstruktur in einem ersten axialen Ankerbereich eines zapfen- oder kielförmigen Ankerabschnitts des des Tibia-Implantats nach Figur 1;
Figuren 4a-4d verdeutlicht schematisch die Erstreckung von sich erhebenden Strukturen bei der in radialer Richtung hinterschnittfreien Oberflächenstruktur in dem ersten axialen Ankerbereich.

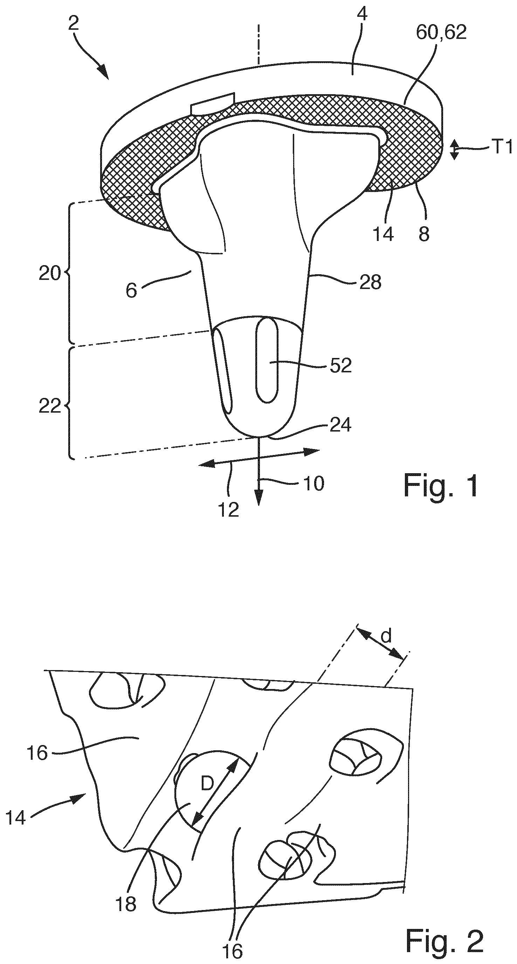

Figur 1 ein erfindungsgemäß ausgebildetes Tibia-Implantat 2 für eine Kniegelenkersatzprothese. Es ist in einem additiven Fertigungsverfahren, insbesondere durch metallischen 3D-Druck, hergestellt und umfasst einen Plateauabschnitt 4 und einen auf der tibiazugewandten Seite des Plateauabschnitts 4 vorspringenden zapfen- oder kielförmigen Ankerabschnitt 6. Der zapfen- oder kielförmige Ankerabschnitt 6 erstreckt sich ausgehend von einer tibiazugewandten Seite 8 des Plateauabschnitts 4 in einer axialen Richtung 10, die auch eine Implantationsrichtung des Tibia-Implantats 2 bildet. Eine hierzu radiale Richtung ist mit Bezugszeichen 12 angedeutet. In nicht dargestellter Weise kann der Plateauabschnitt 4 tibiaabgewandt einen Lagerbereich für artikulierende kondyläre Gelenkoberflächen bilden; insbesondere kann dort ein Meniskusersatzteil in bekannter und daher nicht dargestellter Weise anordenbar sein. Figur 1 zeigt das Tibia-Implantat 2 schräg zur axialen Richtung 10 von unten, also mit Blick auf die tibiazugewandte Seite 8 des Plateauabschnitts 4.

Diese tibiazugewandte und mit Tibiaknochengewebe in Kontakt gelangende Seite 8 des Plateauabschnitts 4 ist mit einer dreidimensional porösen offenporigen Oberflächenstruktur 14 ausgebildet. Diese Oberflächenstruktur 14 weist in der axialen Richtung 10 und in weiteren Richtungen hintergreifbare Brücken, Stege oder Wandbereiche auf. Diese dreidimensional poröse offenporige Oberflächenstruktur 14 ist in Figur 2 stark vergrößert dargestellt, und man erkennt dort eine ein dreidimensionales Gitter bildende Stegstruktur, die aus gitterartig verbundenen Stegen 16 aufgebaut ist, wobei diese Stege 16 in mehreren Richtungen hintergreifbare Brücken, Stege oder Wandbereiche bilden. Diese Oberflächenstruktur 14 und deren Stege 16 sind somit durch einwachsendes Tibiaknochengewebe gewissermaßen in jeder Richtung, also in axialer Richtung 10 und in radialer Richtung 12, hintergreifbar, wodurch eine innige Anbindung von Tibiaknochengewebe an die tibiazugewandte Seite 8 des Plateauabschnitts 4 ausgebildet wird und hierdurch ein stabiler Festsitz des Tibia-Implantats 2 im Tibiaknochen erreicht werden kann. Die in Figur 2 angedeutete Kugel 18 dient zur Andeutung und Bemaßung von Porengrößen innerhalb der dreidimensional porösen Oberflächenstruktur 14. Sie ist nicht Bestandteil der Struktur, sondern dient lediglich der Anschauung. Die Stege 16 der Steg- oder Gitterstruktur führen zu einer ersten Rauigkeit, ausgedrückt in Ra, der Oberflächenstruktur 14. Sie weist eine Tiefenerstreckung T1 ausgehend von einer von außen angelegten und die Oberflächenstruktur berührenden Hüllfläche bis zum dreidimensional dichten metallischen Grund des Plateauabschnitts 4 auf, wie in der Beschreibungseinleitung angegeben. Dasselbe gilt für den Stegdurchmesser d und eine Porengröße D (beides angedeutet in Figur 2).

Ausgehend von der tibiazugewandten Seite 8 des Plateauabschnitts erstreckt sich der zapfen- oder kielförmige Ankerabschnitt in axialer Richtung 10. Er umfasst einen ersten axialen Ankerbereich 20 und daran anschließend einen zweiten axialen Ankerbereich 22, welcher auch ein distales Ende 24 des gesamten zapfen- oder kielförmigen Ankerabschnitts 4 bildet.

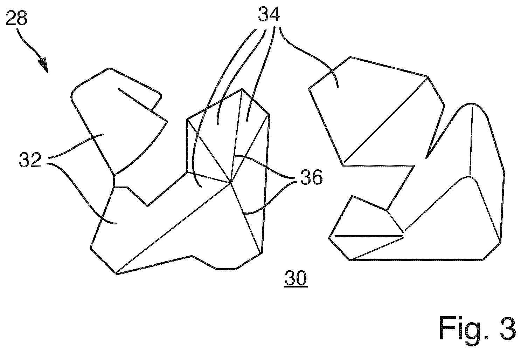

Wie in Figur 1 angedeutet, umfasst der erste axiale Ankerbereich 20 umfangsseitig eine in der radialen Richtung 12 hinterschnittfreie Oberflächenstruktur 28, die frei von hintergreifen Brücken und Brücken bildenden Stegen ist. Der Aufbau dieser in der radialen Richtung 12 hinterschnittfreien Oberflächenstruktur 28 ist stark schematisch in Figur 3 angedeutet, und zwar in der radialen Richtung 12 betrachtet, also mit Blick auf einen Außenumfang des ersten axialen Ankerbereichs 20. In diesem beispielhaft in Figur 3 dargestellten Fall ist die Oberflächenstruktur 28 gebildet durch sich von einer Grundfläche 30 des ersten axialen Ankerbereichs 20 weg erstreckenden oder erhebenden Strukturen 32. Die Grundfläche 30 ist im beispielhaft und bevorzugt dargestellten Fall glatt und gegenüber den Strukturen 32 eben ausgebildet. Die sich erhebenden Strukturen 32 sind im ebenfalls beispielhaft dargestellten Fall von einer Vielzahl von ebenen Facettenflächen 34 gebildet und begrenzt, die über im Wesentlichen gerade Kanten 36 an einander angrenzen. Die Oberflächenstruktur 28 weist eine Tiefenerstreckung T2 (in den Figuren nicht dargestellt) ausgehend von einer von außen angelegten und die Erhebungen 32 berührenden Hüllfläche bis zum dreidimensional dichten metallischen Grund oder der Grundfläche 30 des ersten axialen Ankerbereichs 20 auf, wie in der Beschreibungseinleitung angegeben.

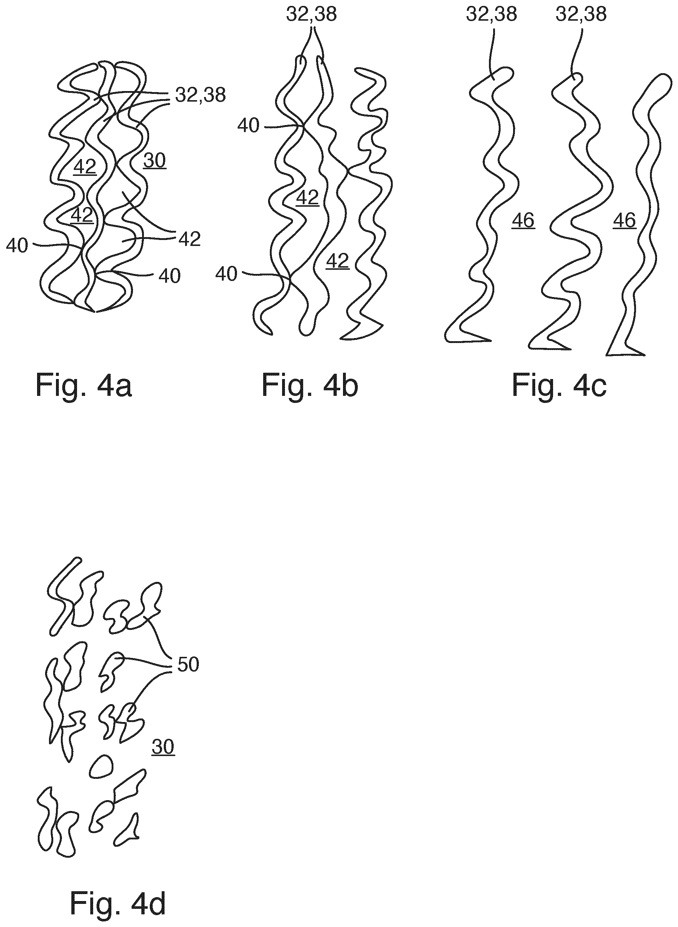

Die Figuren 4a bis 4d zeigen beispielhaft und stark vereinfacht, d.h**.** ohne Andeutung einer dreidimensionalen Struktur, lediglich in zweidimensionaler Aufsicht die Anordnung von sich erhebenden Strukturen 32 der in radialer Richtung 12 hinterschnittfreien Oberflächenstruktur 28. Die Figuren 4a bis 4d verdeutlichen in Aufsicht auf den ersten axialen Ankerbereich 20 in radialer Richtung 12 betrachtet verschiedene Ausführungsformen des Verlaufs und der Erstreckung von sich erhebenden Strukturen 32, wobei die dreidimensionale Gestalt, etwa eine Begrenzung durch Facettenflächen 34, wie in Figur 3 dargestellt, in den Figuren 4a bis 4d weder zwingend vorgesehen noch dargestellt ist. Es geht hier vielmehr um die Anordnung und Erstreckung der sich erhebenden Strukturen bei dem ersten axialen Ankerbereich 20.

In Figur 4a sind sich erhebende Strukturen 32 dargestellt, die hin- und her mäandrierend und damit leporelloförmig oder zickzack-artig ausgebildet sind und hierfür durch Bezugszeichen 38 bezeichnet sind. In Figur 4a bilden nebeneinander angeordnete mäandrierende Strukturen 32 Berührungspunkte 40, so dass zwischen benachbarten Strukturen 32 maschenartig erhebungsfreie oder weniger erhabene Gebiete 42 eingeschlossen oder begrenzt werden. Diese können - wie vorausgehend schon erwähnt - von glatten Bereichen einer Grundfläche 30 des ersten axialen Ankerbereichs 20 gebildet sein.

Bei der Darstellung nach Figur 4b haben benachbarte mäandrierende Strukturen 32, 38 nur eine geringere Anzahl von Berührungspunkten 40, so dass zwischen den Strukturen 32, 38 langgestreckte erhebungsfreie oder weniger erhabene Gebiete 42 begrenzt werden. Es könnte so der Eindruck eines ebenen zerissenen Netzes entstehen.

Bei Figur 4c sind benachbarte mäandrierende sich erhebende Strukturen 32, 38 ohne sich zu berühren derart voneinander beabstandet, dass zwischen ihnen streifenförmig zusammenhängende erhebungsfreie oder weniger erhabene Gebiete 46 gebildet werden.

Schließlich soll Figur 4d eine Oberflächenstruktur 28 verdeutlichen, bei der eine Vielzahl von inselartig angeordneten und voneinander beabstandeten sich erhebenden Strukturen 50 gebildet sind, die sich wiederum insbesondere und vorzugsweise von einer glatten Grundfläche 30 des ersten axialen Ankerbereichs 20 erheben.

Der sich an den ersten axialen Ankerbereich 20 anschließende zweite axiale Ankerbereich 22 weist keine dreidimensional porös strukturierte Oberfläche auf sondern ist glatt ausgebildet und könnte zudem poliert sein. Makroskopische Ausnehmungen 52 sind hiermit nicht gemeint und können vorgesehen sein.

Schließlich sei erwähnt, dass der Plateauabschnitt 4 auch nicht mit Tibiaknochengewebe in Kontakt stehende Oberflächenbereiche 60 aufweist, die Umfangsbereiche des Plateauabschnitts 4 bilden und die im implantierten Zustand in Weichteilkontakt stehen. Diese Oberflächenbereiche 60 sind glatt ausgebildet und können mit einer weiteren Beschichtung 62 versehen sein, welche die Oberflächenrauheit dieser glatten Oberflächenbereiche 60 weiter herabsetzt, wie in der Beschreibungseinleitung und dem in Bezug genommenen Stand der Technik im Einzelnen dargelegt ist.

## Patentansprüche

1. Tibia-Implantat (2) für Gelenkersatz, welches aus Metalllegierung in einem additiven Fertigungsverfahren, insbesondere durch metallischen 3D-Druck, hergestellt ist, mit einem Plateauabschnitt (4), an welchem tibiaabgewandt ein Lagerbereich für artikulierende kondyläre Gelenkoberflächen vorgesehen und insbesondere ein Meniskusersatzteil anordenbar ist, und mit einem auf der tibiazugewandten Seite (8) des Plateauabschnitts (4) vorspringenden und sich davon in einer axialen Richtung (10) wegerstreckenden zapfen- oder kielförmigen Ankerabschnitt (6), welcher in einen hierfür vorbereiteten Kanal in einem Tibiaknochen in der axialen Richtung (10) einsetzbar ist,
**dadurch gekennzeichnet,**
**dass** der Plateauabschnitt (4) auf seiner tibiazugewandten und mit Tibiaknochengewebe in Kontakt gelangenden Seite (8) eine dreidimensional poröse offenporige Oberflächenstruktur (14) mit in der axialen Richtung (10) hintergreifbaren Brücken, Stegen oder Wandbereichen aufweist, wobei die dreidimensional poröse offenporige Oberflächenstruktur (14) eine erste Rauigkeit aufweist, und
**dass** der zapfen- oder kielförmige Ankerabschnitt (6) anschließend an den Plateauabschnitt (4) in einem ersten axialen Ankerbereich (20) umfangsseitig eine in einer radialen Richtung (12) hinterschnittfreie, das heißt von hintergreifbaren Brücken und brückenbildenden Stegen freie Oberflächenstruktur (28) mit einer zweiten Rauigkeit aufweist, die geringer ist als die erste Rauigkeit, und
**dass** der zapfen- oder kielförmige Ankerabschnitt (6) in der axialen Richtung (10) anschließend an den ersten axialen Ankerbereich (20) einen zweiten axial frei endenden axialen Ankerbereich (22) mit einer glatten Oberfläche aufweist.

2. Tibia-Implantat nach Anspruch 1, **dadurch gekennzeichnet, dass** die dreidimensional poröse offenporige Oberflächenstruktur (14) eine Tiefenerstreckung (T1) ausgehend von einer die Oberflächenstruktur (14) einhüllenden Oberfläche von wenigstens 1,5 mm, insbesondere von wenigstens 1,8 mm, insbesondere von wenigstens 2,0 mm, insbesondere von wenigstens 2,5 mm, insbesondere von höchstens 4,0 mm, insbesondere von höchstens 3,8 mm, insbesondere von höchstens 3,5 mm, insbesondere von höchstens 3,0 mm aufweist.

3. Tibia-Implantat nach Anspruch 1 oder 2, **dadurch gekennzeichnet, dass** die dreidimensional poröse offenporige Oberflächenstruktur (14) von einer zusammenhängenden ein dreidimensionales Gitter bildenden Stegstruktur gebildet ist und dass ein Stegdurchmesser (d) wenigstens 0,5 mm, insbesondere wenigstens 0,6 mm insbesondere wenigstens 0,7 mm, insbesondere höchstens 1,1 mm, insbesondere höchstens 1,0 mm, insbesondere höchstens 0,9 mm beträgt.

4. Tibia-Implantat nach Anspruch 1, 2 oder 3, **dadurch gekennzeichnet, dass** die dreidimensional poröse offenporige Oberflächenstruktur (14) eine Porengröße von wenigstens 0,8 mm, insbesondere von wenigstens 0,9 mm, insbesondere von wenigstens 1,0 mm, insbesondere von höchstens 1,4 mm, insbesondere von höchstens 1,3 mm, insbesondere von höchstens 1,2 mm aufweist, wobei es sich hierbei um den Durchmesser (D) einer Kugel handelt, die in den Poren aufnehmbar ist.

5. Tibia-Implantat nach einem oder mehreren der vorstehenden Ansprüche, **dadurch gekennzeichnet, dass** die in radialer Richtung (12) hinterschnittfreie Oberflächenstruktur (28) des ersten axialen Ankerbereichs (20) eine Tiefenerstreckung (T2) ausgehend von einer die Oberflächenstruktur (28) einhüllenden Oberfläche von wenigstens 0,2 mm, insbesondere von wenigstens 0,3 mm und von höchstens 0,9 mm, insbesondere von höchstens 0,8 mm, insbesondere von höchstens 0,7 mm aufweist.

6. Tibia-Implantat nach einem oder mehreren der vorstehenden Ansprüche, **dadurch gekennzeichnet, dass** die in radialer Richtung (12) hinterschnittfreie Oberflächenstruktur (28) des ersten axialen Ankerbereichs (20) sich von einer Grundfläche (30) des Ankerbereichs (20) erhebende Strukturen (32, 38, 50) aufweist.

7. Tibia-Implantat nach einem oder mehreren der vorstehenden Ansprüche, **dadurch gekennzeichnet, dass** die in radialer Richtung (12) hinterschnittfreie Oberflächenstruktur (28) des ersten axialen Ankerbereichs (20) bei Betrachtung in radialer Richtung (12) hin- und her mäandrierende sich erhebende Strukturen (32, 38) aufweist.

8. Tibia-Implantat nach Anspruch 6 oder 7, **dadurch gekennzeichnet, dass** die sich erhebenden Strukturen (32, 38, 50) eine Vielzahl von ebenen Facettenflächen (34) umfassen, die über Kanten (36) aneinander angrenzen.

9. Tibia-Implantat nach Anspruch 7 oder 8, **dadurch gekennzeichnet, dass** die mäandrierenden sich erhebenden Strukturen (32, 38) im Wesentlichen ebene oder glatte Bereiche einer Grundfläche (30) zwischen sich begrenzen.

10. Tibia-Implantat nach Anspruch 7, 8 oder 9, **dadurch gekennzeichnet, dass** benachbarte mäandrierende sich erhebende Strukturen (32, 38) einander berühren und zwischen sich erhebungsfreie oder weniger erhabene Gebiete (42) begrenzen.

11. Tibia-Implantat nach Anspruch 7, 8 oder 9, **dadurch gekennzeichnet, dass** benachbarte mäandrierende sich erhebende Strukturen (32, 38) voneinander beabstandete sind, so dass zwischen ihnen streifenförmig zusammenhängende erhebungsfreie oder weniger erhabene Gebiete (46) begrenzt werden.

12. Tibia-Implantat nach einem oder mehreren der vorstehenden Ansprüche, **dadurch gekennzeichnet, dass** die in radialer Richtung (12) hinterschnittfreie Oberflächenstruktur (28) des ersten axialen Ankerbereichs (20) eine Vielzahl von einzelnen inselartig voneinander beabstandeten sich erhebenden Strukturen (50) aufweist.

13. Tibia-Implantat nach einem oder mehreren der vorstehenden Ansprüche, **dadurch gekennzeichnet, dass** es aus Titan oder Titan-Legierung gefertigt ist.

14. Tibia-Implantat nach einem oder mehreren der vorstehenden Ansprüche, **dadurch gekennzeichnet, dass** nicht mit Tibiaknochengewebe in Kontakt stehende Oberflächenbereiche (60) des Plateauabschnitts (4), also in Weichteilkontakt stehende Umfangsbereiche oder mit einem Meniskusersatzteil in Kontakt stehende Oberflächenbereiche, glatt sind.

15. Tibia-Implantat nach einem oder mehreren der vorstehenden Ansprüche, **dadurch gekennzeichnet, dass** nicht mit Tibiaknochengewebe in Kontakt stehende glatte Oberflächenbereiche (60) des Plateauabschnitts (4) zumindest bereichsweise eine Beschichtung (62) aufweisen, mittels derer eine Oberflächenrauheit dieser glatten Oberflächenbereiche (60) weiter herabgesetzt wird.

16. Tibia-Implantat nach Anspruch 15, **dadurch gekennzeichnet, dass** die Beschichtung (62) eine keramische Oberfläche, insbesondere aus oder auf Basis von Zirkonnitrid, umfasst.

17. Tibia-Implantat nach Anspruch 15 oder 16, **dadurch gekennzeichnet, dass** die Beschichtung (62) mehrlagig ausgebildet ist und über eine Haftvermittlungschicht, insbesondere auf Kobalt-Chrom-Basis oder auf Titan-Basis, an das Implantatteil angebunden ist

18. Tibia-Implantat nach Anspruch 15, 16 oder 17, **dadurch gekennzeichnet, dass** die Beschichtung (62) Schichten auf Basis von Kobalt-Chrom und/oder auf Basis von Chromnitrid (CrN) und/oder auf Basis von Chromcarbonitrid (CrCN) und/oder auf Basis von Zirkoniumnitrid (ZrN) umfasst.

19. Tibia-Implantat nach Anspruch 15, 16, 17 oder 18, **dadurch gekennzeichnet, dass** die Beschichtung (62) eine Deckschicht auf Basis von Zirkonnitrid und innere Schichten auf Basis von Chromnitrid (CrN) oder Chromcarbonitrid (CrCN) aufweist, wobei sich innere Schichten auf Basis von Chromnitrid (CrN) und Chromcarbonitrid (CrCN) abwechseln können.
